# EUROPEAN PATENT SPECIFICATION

(11) **EP 3 965 769 B1**
(45) Date of publication and mention of the grant of the patent: **07.09.2022**
(21) Application number: 20729634.4
(22) Date of filing: 08.05.2020
(51) Int. Cl.: A61K 31/517, A61K 45/06, A61P 25/16, A61P 25/28

(54) **USE OF 2-PHENYL-6-(1H-IMIDAZOL-1-YL)QUINAZOLINE FOR TREATING NEURODEGENERATIVE DISEASES, PREFERABLY ALZHEIMER'S DISEASE**
VERWENDUNG VON 2-PHENYL-6-(1H-IMIDAZOL-1-YL)CHINAZOLIN ZUR BEHANDLUNG VON NEURODEGENERATIVEN ERKRANKUNGEN, INSBESONDERE MORBUS ALZHEIMER
UTILISATION DE QUINAZOLINE 2-PHÉNYL-6-(1H-IMIDAZOL-1-YL) POUR LE TRAITEMENT DE MALADIES NEURODÉGÉNÉRATIVES, DE PRÉFÉRENCE LA MALADIE D'ALZHEIMER

(30) Priority: 10.05.2019 EP 19173783
(43) Date of publication of application: 16.03.2022
(73) Proprietor: Rottapharm Biotech S.r.l., 20900 Monza (IT)
(72) Inventor: ROVATI, Lucio Claudio, 20900 Monza (IT); CASELLI, Gianfranco, 20129 Milano (IT); SALA, Emanuele, 20862 Arcore (IT)
(74) Representative: Cattaneo, Elisabetta
(86) International application number: PCT/EP2020/062813
(87) International publication number: WO 2020/229329

(56) References cited:
- WO-A1-2009/152868
- GRIÑÁN-FERRÉ CHRISTIAN ET AL: "Behavioral and Cognitive Improvement Induced by Novel Imidazoline I2Receptor Ligands in Female SAMP8 Mice", NEUROTHERAPEUTICS, ELSEVIER INC, US, vol. 16, no. 2, 20 November 2018 (2018-11-20), pages 416-431, XP036801489, ISSN: 1933-7213, DOI: 10.1007/S13311-018-00681-5 [retrieved on 2018-11-20]

## Description

### FIELD OF THE INVENTION

The present invention provides 2-phenyl-6-(1H-imidazol-1-yl)quinazoline to treat neurodegenerative diseases, wherein the neurodegenerative disease is a disease selected from the group consisting of Alzheimer's disease, Lewy body dementia, frontotemporal dementia, amyotrophic lateral sclerosis, Huntington disease, prion diseases and HIV-associated dementia.

### BACKGROUND OF THE INVENTION

Neurodegenerative diseases (ND) are a growing cause of mortality and morbidity worldwide, particularly in the elderly. They comprise highly diffused pathologies such as Alzheimer's disease, Parkinson's disease, Lewy body dementia, frontotemporal dementia, amyotrophic lateral sclerosis, Huntington disease, and prion diseases, with a particular focus on similarities and differences among these syndromes.

The neurodegenerative diseases represent a great challenge for basic science and clinical medicine because of their prevalence, complex biochemistry and pathology. Therefore new mechanism related treatment represent an unmet medical need.

Alzheimer's disease (AD) is the most common type of dementia associated with progressive cognitive decline and memory loss. It is the sixth-leading cause of death in the United States and will afflict an estimated 14 million people in this country by 2050.

Current treatments for AD provide little symptomatic benefit, and the most recent approval by the US Food and Drug Administration occurred in 2003. Since then, more than 400 clinical trials of therapeutics for AD have been registered, with a failure rate of nearly 100% in those trials for which results have been reported. The lack of progress in the treatment and prevention of AD, mainly targeting the β-amyloid protein or the paired helical filament tau protein that accumulates in the brain of a person with AD, is frustrating and there is the need for a change of perspective, searching for new pathways and targets to get new and effective disease-modifying agents.

AD is typified by distinct tissue changes associated with accumulation of extracellular amyloid-β (Aβ) peptides, derived from the cleavage of amyloid precursor protein (APP), and intracellular deposits of hyperphosphorylated tau. Aβ and tau aggregates are neurotoxic and trigger neurodegenerative processes in the brain, suggesting that Aβ and tau are central for driving AD pathogenesis. Furthermore, many genes that affect risk for AD are expressed in microglia (Zhang B, Cell, 2013), and during ageing and the stage of the disease, microglia and astrocytes change their activation phenotype. These findings raise the possibility that innate immune activation may actively contribute to AD pathogenesis. In the presence of harmful stimuli, including misfolded proteins such as Aβ, microglial cells mount an acute immune response in the brain. If the response does not resolve, the chronic activation of microglia and the recruitment of astrocytes divert their physiological and beneficial functions. Imidazol(ine)/guanidine compounds elicit central and peripheral effects through the interaction with non-adrenoreceptor sites, the so-called imidazoline receptors (Escribá P, Ann. N. Y. Acad. Sci., 1999) which have been classified into two main types: I1 binding sites (BS) subtype, identified by drugs such as clonidine and involved in blood pressure regulation; I2 binding sites, identified firstly by idazoxan (mixed I2BS and α2-adrenergic receptors ligand) and characterized by selective ligands devoid of 11-IBS and α2-adrenergic receptors affinity (e.g. 2-BFI, BU224). In addition an I3BS was identified as a typical imidazoline subtype present in pancreatic beta-cells and involved in insulin secretion and recognized by efaroxan.

One of the endogenous ligands for imidazoline receptors is agmatine, an amine intermediate in polyamine biosynthesis. Agmatine is widely distributed in the body and possibly acts as neurotransmitter and/or neurotransmodulator in the brain. Besides imidazoline receptors, agmatine also binds to other target receptors such as α2-adrenergic, N-methyl-D-aspartate (NMDA) and serotonin receptors with lower affinity to produce physical functions.

I2BS are widely distributed and in the brain are expressed on neurons, but mainly on glial cells (Ruggiero DA, Brain Res., 1998), located principally in the outer membrane of mitochondria.

Both neuroprotective and anti-inflammatory actions of imidazoline drugs have been reported (Regunathan S, Ann. N. Y. Acad. Sci., 1999), but the I2BS pharmacology is still elusive. I2 receptors represent a group of binding sites harboured on various proteins, whose nature and biological significance remain unclear (Escribá P, Ann. N. Y. Acad. Sci.,1999).

Some experimental evidence collected during last 3 decades show that I2 ligands may exert at least a partial neuroprotective effects with different mechanisms and in different models of neurodegeneration.

Chronic imidazoline drug treatment has long been established to increase GFAP expression in astrocytes (Olmos G, Br. J. Pharmacol., 1994).

In astrocytes and macrophages, idazoxan is able to inhibit inducible NOS (iNOS) activity and thereby reduce levels of NO-mediated neurotoxicity (Feinstein D, Mol. Pharmacol., 1999).

BU224 (selective I2 ligand) has been demonstrated to down-regulate pro-apoptotic factors in rat brain cortex, and as such, may mediate neuroprotective actions by inhibition of key components of canonical apoptotic signalling in the brain (Garau C J, Psychopharmacol., 2013).

2-BFI (selective I2 ligand) shows neuroprotective effect both in vitro and in vivo models of ischemic stroke. In vitro 2-BFI prevents lipid peroxidation and mitochondrial apoptosis in an astrocyte oxygen-glucose deprivation (Tian J, J.Neurosci. Res., 2018), whereas in a brain injury induced by middle cerebral artery occlusion, a rat model of transient cerebral ischemia, 2-BFI induces Bcl-2 expression (a gene with key role in neuron survival during cerebral ischemia) (Han Z, Brain Res., 2010) and protects the blood-brain barrier integrity, reducing matrix metalloprotease 9 (MMP-9) expression and upregulating tight junction proteins and collagen IV (Zhang ZJ, Stroke Cerebrovasc. Dis.,2018).

I2 receptor ligands bind with low affinity the NMDA receptor and modulate its activity in a non competitive and reversible manner, similarly to memantine reducing NMDA-mediated glutamate toxicity in vitro and in vivo (Jiang SX, Eur. J. Pharmacol., 2010). I2 imidazoline drugs inhibit Aβ-induced neuronal toxicity by reducing Erk1/2 activation (Montolio M, J. Med. Chem., 2012).

Chronic treatment with 2-BFI attenuates Experimental Autoimmune Encephalomyelitis (a mouse model of multiple sclerosis) restoring B-CK and CaATPase enzymatic activities and maintaining calcium-dependent calpain activation at the basal levels (Wang P, Biochem. Biophys. Res. Commun., 2011), decreasing levels of pro-inflammatory cytokines IL-17A and IFN-gamma and increasing level of anti-inflammatory cytokine IL-10 (Zhu YB, Neurochem. Res., 2015).

In an AD model (by injection of Aβ1-42 in rat hippocampus), 2-BFI ameliorates the learning and memory abilities, lowering oxidative stress, down-regulating the release of inflammatory factors and inhibiting neural cells apoptosis (Tian JS, J. Integr. Neurosci., 2017).

BU224 is partly neuroprotective against kainic acid-induced excitotoxic signalling (Keller BJ, Psychopharmacol., 2016).

Acute treatment with BU224 increases in the hippocampus p-FADD/FADD ratio (an index of cell survival) and reduces p35 cleavage into neurotoxic p25 (Abás S, ACS Chem. Neurosci., 2017).

Treatment with agmatine, the endogenous imidazoline receptor ligand, significantly improves cognitive functions, recovering memory deficits induced in diabetic rats (Bhutada P, Prog. Neuro-Psychopharmacology Biol. Psychiatry 2012).

On the other hand, increases in the density of I2Rs using radioligand binding assays have been observed during aging and in AD brains, probably due to their location in astrocytes (García-Sevilla, Neurosci. Lett., 1998).

Protein kinase C (PKC) is a phospholipid-dependent family of Serine/Threonine protein kinases that comprise an extensive signaling net in the brain. Molecular cloning studies have revealed 12 PKC isozymes, which are divided into three subgroups: (1) classical PKCs, (2) novel PKCs, and (3) atypical PKCs. PKC isoforms play a key role in various cognitive functions including learning and memory. In particular, PKCε, classified as a novel PKC (not requiring calcium for their activation), is a phorbol ester/diacylglycerol (DAG)-sensitive and calcium-independent serine/threonine kinase. It is a key regulator of various signal-transducing events, and therefore its demand to be present in several subcellular locations is met by translocation of the kinase by isozyme-specific chauffeur proteins. Aberrant translocation of the kinase could miscue the signaling outputs and hence be detrimental to cellular physiology. Due to this complex biology, its role in neurodegenerative disorders is still controversial. While some papers suggest that inhibiting PKCε activation results in an overall protection against neurodegenerative diseases (for instance: in Alzhemeir's disease Zara S, Brain Research 2011; in ischemia induced neurodegeneration, Kumar V, J Neuro Res. 2019), the majority of papers in the past pointed to a protective role for PKCε activation in these diseases. This concept led to the proposal of an activator of this kinase (Bryostatin-1) as a clinical candidate for Alzheimer's disease, for example in US 2008/0004332 and in US 2016/0025704. In the former it is stated that an inhibitor of PKC in peripheral tissues could be associated to the PKC activator just to dampen the possible side-effects induced by activating PKC in the peripheral tissues, where "peripheral tissues means tissues other than brain". Unfortunately, in May 2017 it has been communicated that, in the Phase II proof of the concept study, this approach using the PKCε Bryostatin-1 failed to meet the primary endpoint, which measured improvements in Severe Impairment Battery (SIB) scores vs. placebo. GRIÑÁN-FERRÉ CHRISTIAN ET AL: "Behavioral and Cognitive Improvement Induced by Novel Imidazoline I2-Receptor Ligands in Female SAMP8 Mice", NEUROTHERAPEUTICS, vol. 16, no. 2, 20 November 2018, pages 416-431 discloses the use of two specific I2 imidazoline ligands MCR9 and MCR5 in a model of Alzheimer's disease.

### SUMMARY OF THE INVENTION

The inventors found out that the compound of formula 2-phenyl-6-(1H-imidazol-1-yl)quinazoline (named also as CR4056) can be used for treating a neurodegenerative disease , wherein the neurodegenerative disease is a disease selected from the group consisting of Alzheimer's disease, Lewy body dementia, frontotemporal dementia, amyotrophic lateral sclerosis, Huntington disease, prion diseases and HIV-associated dementia.

This molecule combines 1) a strong activity towards I2 binding sites, 2) a long lasting inhibition of the translocation of PKCε towards the cell membrane in neurons, and 3) a striking ability to cross the blood brain barrier. The combination of these three features results in a surprising efficacy in models of memory impairment and of Alzheimer's disease for CR4056. This resulted to be particularly innovative since the prior art explicitly excludes the use of brain penetrant PKC inhibitors in the treatment of neurodegenerative diseases.

In a first aspect therefore the invention concerns a compound of formula 2-phenyl-6-(1H-imidazol-1-yl)quinazoline or a pharmaceutically acceptable salt thereof for use in the treatment of a neurodegenerative disease, wherein the neurodegenerative disease is a disease selected from the group consisting of Alzheimer's disease, Lewy body dementia, frontotemporal dementia, amyotrophic lateral sclerosis, Huntington disease, prion diseases and HIV-associated dementia.

When in the present invention, reference is made to a neurodegenerative disease, it is intended a disease selected from the group of chronic, progressive disorders characterized by the gradual loss of neurons in discrete areas of the central nervous system (CNS).

The neurodegenerative disease according to the invention is a disease selected from the group consisting of Alzheimer's disease, Lewy body dementia, frontotemporal dementia, amyotrophic lateral sclerosis, Huntington disease, prion diseases and HIV-associated dementia, preferably Alzheimer's disease.

The mechanism(s) underlying the progressive nature of such a neurodegenerative disease remains unknown but a timely and well-controlled inflammatory reaction is essential for the integrity and proper function of the CNS.

The compound 2-phenyl-6-(1H-imidazol-1-yl)quinazoline (named also as CR4056) is a first-in-class imidazoline-2 receptor ligand characterized by potent analgesic activity in different animal models of inflammatory, neurogenic, neuropathic, postoperative, fibromyalgia-like, and osteoarthritis pain (WO2008014822 A1, WO2009152868 A1, Ferrari F, JPainRes, 2011; Lanza M, B. J. of Pharmacol., 2014).

Moreover, CR4056 is endowed with a long-lasting (but still reversible) ability to inhibit the translocation of PKCε to the cell membrane of primary neurons triggered by inflammatory stimuli. This long-lasting activity is characteristic of CR4056 and it is not shared by other anti-inflammatory or analgesic compounds. Yet, this activity is idazoxan (a prototypical I2 receptor antagonist) resistant and seems, therefore, independent from the classical pathway induced by I2 ligands. Without being bound by any theory, the inventors deemed that the inhibition of PKCε translocation to the plasma membrane in neurons triggered by inflammatory stimuli could be a surprisingly better target to control neurodegeneration and found out that CR4056 was the best candidate to do it.

Finally, CR4056 is endowed with a peculiar ability to cross the blood-brain barrier and to target the Central Nervous System: the presence of CR4056 in the brain was studied in rats 1h after single oral administration at a dose of 30 mg/kg suspended in 0.5% Methocel. The mean levels of CR4056 in the brain were 32272 ng/g. Mean brain/plasma ratio was 11.8, suggesting a marked ability of CR4056 to concentrate in the brain.

The inventors tested CR4056 in *in vitro* and *in vivo* models (transgenic or pharmacological) for neurodegenerative diseases, specifically for Alzheimer's disease and found out that, surprisingly, CR4056 significantly reduced microglia activation, thus contributing to the neuroprotection, it significantly improved the cognitive performance and was able to reverse the memory impairment in both transgenic and pharmacological models of Alzheimer.

From a safety point of view, even at suprapharmacological concentrations and considering the particular tropism of CR4056 for brain, CR4056 did not show central adverse effect in rats. In particular neither neurobehavioral changes (assessed by the Irwin test) nor impairment of motor coordination and locomotor activity (assessed by rotarod and open field tests) were observed. These findings indicate that CR4056 could be a safe drug for treating neurodegenerative diseases, and, in particular, Alzheimer disease.

### DESCRIPTION OF THE FIGURES

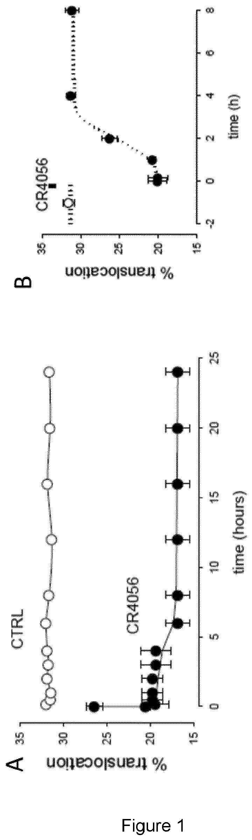

Figure 1 reports the time-course (A) and washout (B) of CR4056 effect on PKCε translocation induced by bradykinin (BK 1µM). In Panel A, open symbols represent the percentage of neurons positive for PKCε translocation following BK stimulation in absence of the drug. Filled symbols represent neurons pre-incubated with CR4056 (10µM) for different times before the stimulation for 30 seconds with BK+CR4056. The first filled symbol indicates the effect of CR4056 co-incubated for 30 seconds with BK, the second filled symbol indicates 10 seconds of pre-incubation of CR4056, and the last indicates 24 hours of pre-incubation of CR4056. The effect of CR4056 was highly significant at all times (t-test, p<0.05, n=6). In Panel B, BK-induced PKCε translocation was evaluated before (open symbol) and after pre-incubation of CR4056 (10µM) at different time intervals (from 10 minutes to 8 hours) (filled symbols). The first datapoint was obtained at the end of the 10 minute pre-incubation, when CR4056 was still present. The subsequent datapoints were obtained after extensive rinsing to ensure complete removal of the drug from extracellular solution (n=3).

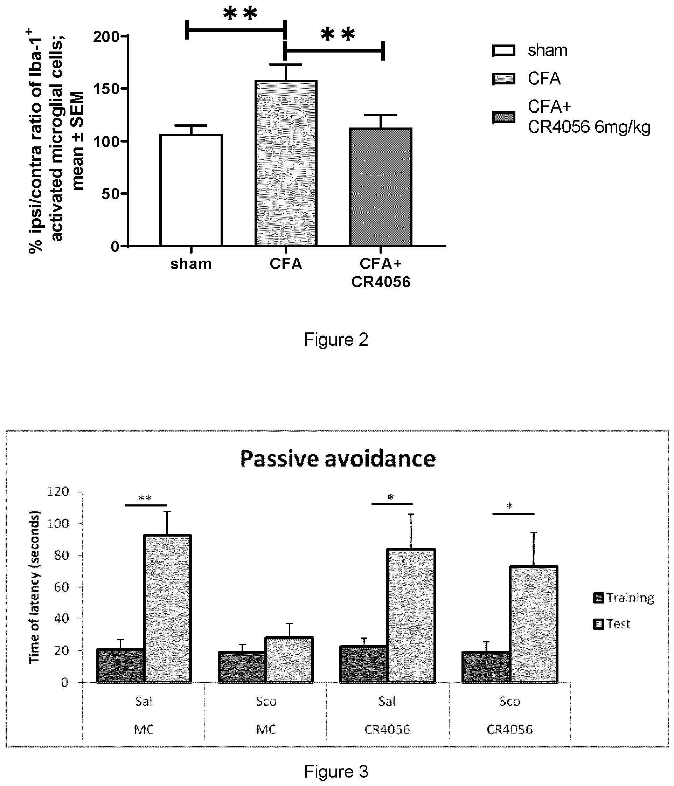

Figure 2 reports the results of spinal microglia activation at 72 hours following CFA injection. The quantification of microglia activation in ipsilateral L5 SC dorsal horn was determined as the number of Iba1-positive microglia, displaying a clearly swollen cell body with reduced processes, within the analyzed selected frame of the superficial laminae. Data represent the mean of 5 animals/group. Ordinary one way ANOVA followed by Dunnet's multiple comparison (**p< 0.05).
Figure 3 reports results of a passive avoidance test in the model of scopolamine-induced memory loss. Rats treated with scopolamine (Sco, 1 mg/kg) or physiological solution (Sal) received CR4056 10 mg/kg bid or its vehicle (MC). t student test. *p<0.05; **P<0.01 (n=6).

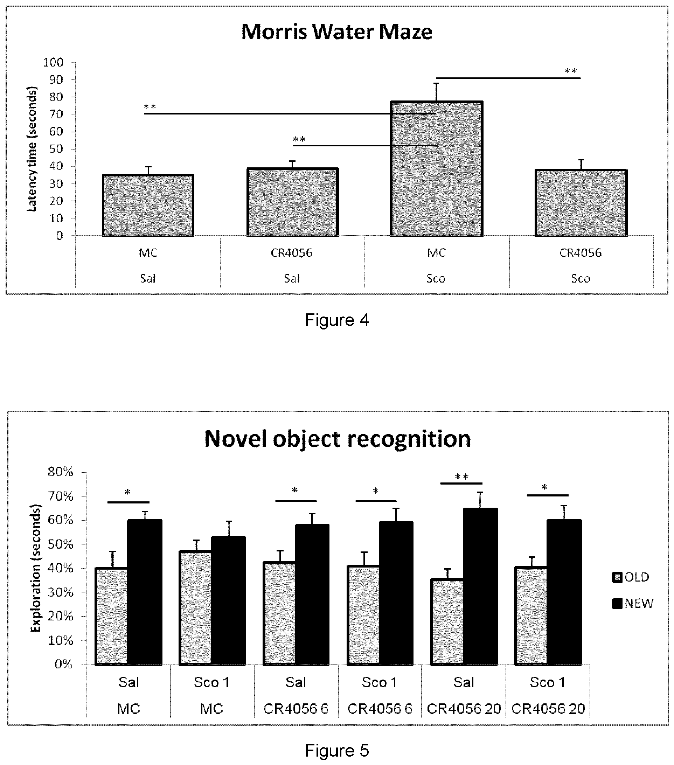

Figure 4 reports the results of the Morris Water Maze test in the model of scopolamine-induced memory loss. Rats treated with scopolamine (Sco, 1 mg/kg) or physiological solution (Sal) received CR4056 20 mg/kg/die or its vehicle (MC). Data represented the mean time spent to reach the hidden platform over four days. Two way ANOVA. Tukey Comparison test. ** P<0.01.
Figure 5 reports the results of Novel Object recognition test in the model of scopolamine-induced memory loss. Data presented as % of time spent on exploration of new and old object in testing phase. *p<0.05; **P<0.01; t student test (n=6).

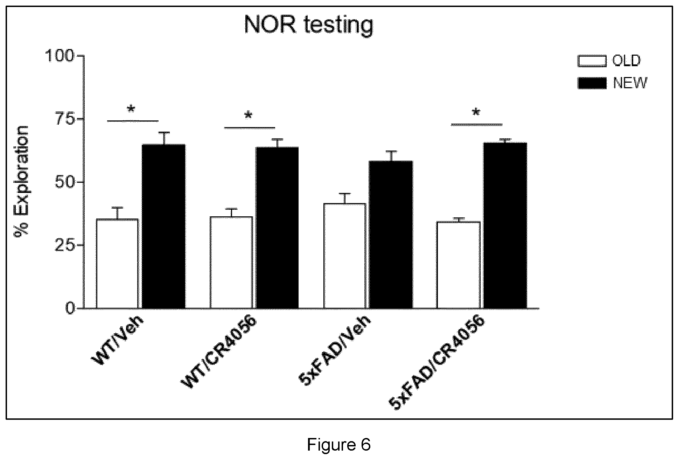

Figure 6 reports the results of Novel Object Recognition test in transgenic 5XFAD mice model of Alzheimer disease. (Obj C = new object) *p<0.0001, unpaired t Student test.

### DETAILED DESCRIPTION OF THE INVENTION

The inventors found out that the compound 2-phenyl-6-(1H-imidazol-1-yl)quinazoline (named also as CR4056) can be used for treating a neurodegenerative disease, wherein the neurodegenerative disease is a disease selected from the group consisting of Alzheimer's disease, Lewy body dementia, frontotemporal dementia, amyotrophic lateral sclerosis, Huntington disease, prion diseases and HIV-associated dementia.

In a first aspect therefore the invention concerns a compound of formula 2-phenyl-6-(1H-imidazol-1-yl)quinazoline or a pharmaceutically acceptable salt thereof for use in the treatment of a neurodegenerative disease, wherein the neurodegenerative disease is a disease selected from the group consisting of Alzheimer's disease, Lewy body dementia, frontotemporal dementia, amyotrophic lateral sclerosis, Huntington disease, prion diseases and HIV-associated dementia.

When in the present invention, it is referred to a neurodegenerative disease, it is intended a disease selected from the group of chronic, progressive disorders characterized by the gradual loss of neurons in discrete areas of the central nervous system (CNS).

A neurodegenerative disease is a disease selected from the group consisting of Alzheimer's disease, Lewy body dementia, frontotemporal dementia, amyotrophic lateral sclerosis, Huntington disease, prion diseases and HIV-associated dementia, preferably Alzheimer's disease.

The mechanism(s) underlying the progressive nature of such a neurodegenerative disease remains unknown but a timely and well-controlled inflammatory reaction is essential for the integrity and proper function of the CNS.

The invention further provides CR4056 for use in a method for treating or preventing the development of a neurodegenerative disease in a subject in need thereof, said method comprising administering a therapeutically effective amount of a compound or a pharmaceutical composition according to the present invention to the subject, thereby treating or reducing the risk of developing a neurodegenerative disease, wherein the neurodegenerative disease is a disease selected from the group consisting of Alzheimer's disease, Lewy body dementia, frontotemporal dementia, amyotrophic lateral sclerosis, Huntington disease, prion diseases and HIV-associated dementia.

According to the invention said neurodegenerative disease is a disease selected from the group consisting of Alzheimer's disease, Lewy body dementia, frontotemporal dementia, amyotrophic lateral sclerosis, Huntington disease, prion diseases and HIV-associated dementia, preferably Alzheimer's disease.

The compound 2-phenyl-6-(1H-imidazol-1-yl)quinazoline can be used as free base or in a salt form. Preferably, the pharmaceutically acceptable salt is a salt chosen from hydrochloride, hydrobromide, hydrogensulphate and sulphate, maleate, fumarate, oxalate, methanesulfonate, succinate, ascorbate, tartrate, acetate, salicylate, citrate, aspartate, ethylenediaminotetraacetate, benzoate and glutamate. Further examples of pharmaceutically acceptable salts are reported in S.M. Berge et al, J.Pharm. Sci. 1977, 66,2.

The compound 2-phenyl-6-(1H-imidazol-1-yl)quinazoline can be also in a polymorphic form or as an hydrate form as described in EP2438058A.

In a second aspect the invention concerns a pharmacological composition comprising the compound 2-phenyl-6-(1H-imidazol-1-yl)quinazoline or a pharmaceutically acceptable salt thereof and a carrier for use in the treatment of a neurodegenerative disease, wherein the neurodegenerative disease is a disease selected from the group consisting of Alzheimer's disease, Lewy body dementia, frontotemporal dementia, amyotrophic lateral sclerosis, Huntington disease, prion diseases and HIV-associated dementia.

Preferably the composition of the invention is used for treating Alzheimer's disease. The composition for use can comprise also pharmaceutically acceptable excipients and can be administered in a pharmaceutical form suitable for the desired administration route.

Pharmaceutically acceptable additives can be excipients, ligands, dispersing agents, colourants, humectants, commonly used for the preparation of tablets, capsules, pills, solutions, suspensions, emulsions for oral administration. Injectable solutions are also contemplated for parental administration, comprising subcutaneous, spinal and transdermal administration.

The pharmaceutical composition according to the present invention is preferably for intravenous, oral, transdermal, intrathecal, intranasal, intraperitoneal or intramuscular administration.

The pharmaceutical compositions according to the present invention can be used alone or in combination with or can comprise one or more further drugs. These drugs can be drugs known for the treatment of a neurodegenerative disease, wherein the neurodegenerative disease is a disease selected from the group consisting of Alzheimer's disease, Lewy body dementia, frontotemporal dementia, amyotrophic lateral sclerosis, Huntington disease, prion diseases and HIV-associated dementia.

The composition for use in treating the neurodegenerative disease can comprise 2-phenyl-6-(1H-imidazol-1-yl)quinazoline (CR4056) or a pharmaceutically acceptable salt in an amount from 15 to 250, mg with respect to the unitary dosage form, resulting in a daily intake from 15 to 500 mg.

The present invention also relates to a combined preparation comprising CR4056 or a pharmaceutically acceptable salt thereof and at least one of NMDA (N-methyl-D-aspartic acid) receptor antagonists and/or acetylcholinesterase inhibitors for simultaneous, sequential or separated use in the treatment of a neurodegenerative disease, wherein the neurodegenerative disease is a disease selected from the group consisting of Alzheimer's disease, Lewy body dementia, frontotemporal dementia, amyotrophic lateral sclerosis, Huntington disease, prion diseases and HIV-associated dementia, preferably Alzheimer's disease.

CR4056 is to be administered at the dosages indicated above, optionally combined with medications belonging to classes of drugs currently approved to treat Alzheimer's disease cognitive symptoms, i.e. NMDA (N-methyl D-aspartic acid) receptor antagonists and acetylcholinesterase inhibitors.

In particular, NMDA receptor antagonist is memantine and the acetylcholinesterase inhibitor is selected from donepezil, rivastigmine and galantamine.

Dosing for memantine (Namenda^{®} or Ebixa^{®}), donepezil (Aricept^{®}), rivastigmine (Exelon^{®}), and galantamine (Razadyne^{®} or Reminyl^{®}) are in accordance to the manufacture's recommendations, respectively Namenda Label Information (2007), Aricept Label Information, Exelon Label Information (2006), and Razadyne Label Information (2008).

The invention will be now described with reference to examples by using *in vitro* and *in vivo* models (transgenic and not) for neurodegenerative diseases.

### EXPERIMENTAL PART

### EXAMPLE 1: IN VITRO EFFECTS OF 2-PHENYL-6-(1H-IMIDAZOL-1-YL)QUINAZOLINE ON THE EXPRESSION OF INFLAMMATORY GENES

### METHODS

A model of astrocytes, the human glioblastoma astrocytoma cell line U373 MG (Uppsala), was used for these experiments. Adherent cells were grown in DMEM medium supplemented with 10% FBS at 37°C with CO₂. 72 hours after plating, cells were treated for 1h with 2-phenyl-6-(1H-imidazol-1-yl)quinazoline (CR4056) (10µM) prepared according to EP2066653 and then stimulated with the pro-inflammatory cytokine IL-1β (2ng/mL) for further 6 and 24h.

At the end of incubation period, total RNA was obtained and retro-transcribed using the High-Capacity cDNA Reverse Transcription Kit (Thermo Fisher Scientific). The levels of expression of COX-2, IL-1β, IL-6 and TNFα were evaluated by RT-PCR analysis, performed using the Applied Biosystems 7500 Fast Real-Time PCR System using specific TaqMan assays and, as an endogenous control, the 18S Pre-Developed TaqMan^{®} Assay (Thermo Fisher Scientific). The data analysis, with normalisation on 18S amplified values, was according to Thermo Fisher Scientific specific instructions for gene expression relative quantification. All individual data were the result of at least three different measurements for each sample.

### RESULTS

2-Phenyl-6-(1 H-imidazol-1-yl)quinazoline (CR4056), after incubation period of 6 hours, reduces COX2 and IL-1β gene expression having an inhibitory effect of 45% on COX2 and 20% on IL-1β gene expression. At this time point, the gene expression of IL-6 and TNFα appeared not yet modulated by CR4056.

After 24h of stimulation, CR4056 reduced the gene expression of all inflammatory markers analyzed having an inhibitory effect of 48% on COX2, 29% on IL-1β, 39% on IL-6 and 52% on TNFα gene expression, as reported in the table below.

**Table I.**

| **Target gene** | **6h** | **24h** |
|---|---|---|
| **COX-2** | 45% | 48% |
| **IL-1b** | 20% | 29% |
| **IL-6** | Not modulated | 39% |
| **TNF-α** | Not modulated | 52% |

Percentage of gene expression inhibition observed in U373 cells stimulated with IL-1β 2 ng/ml and exposed to10 µM CR4056 for the indicated time.

### CONCLUSIONS

The above reported results showed that CR4056 has a modulatory effect on the production of pro-inflammatory cytochines in an astrocytoma cell line. This effect could contribute to the neuroprotective effect.

### EXAMPLE 2: IN VITRO EFFECT OF 2-PHENYL-6-(1H-IMIDAZOL-1-YL) QUINAZOLINE (CR4056) ON PKCε TRANSLOCATION TO THE PLASMA MEMBRANE IN CULTURED NEURONS

### METHODS

Rat dorsal root ganglia (DRGs) were obtained from freshly isolated spines after carefully removing nerve trunks and connective tissue. Larger ganglia, chopped into 2-4 smaller pieces were then incubated for 1 hour at 37°C in 0.125% collagenase (Worthington, Freehold, NJ) dissolved in Dulbecco's modified Eagle's medium (DMEM) containing 10% fetal bovine serum (FBS) plus 1% penicillin/streptomycin and 1% L-glutamine (Euroclone, Milan, Italy). After enzymatic digestion, ganglia were mechanically dissociated and neurons plated at a density such that neurons would cover about 30% of coverslip surface in a single layer, in petri dishes containing wells with a glass bottom coverslip (pre-coated with 10 µg/mL poly-L-lysine and 20 µg/ml laminin, Sigma-Aldrich, Milan, Italy). Cells were incubated for 2-3 days in DMEM as described above, plus 1.5 µg/ml cytosine 1-d-arabinofuranoside (ARA-C, Sigma-Aldrich) to slow down proliferation of non neuronal cells, and 100 ng/ml nerve growth factor (NGF, Sigma-Aldrich) to increase cell health and the and the expression of receptors which are linked to PKCε translocation upon stimulation. Activation of membrane receptors coupled to phospholipase C pathway leads to PKCε translocation from cytoplasm to the plasma membrane. In order to study PKCε behaviour a well established technique was employed (Vellani V, Neuroscience, 2006). This technique involves activation of PKCε rapidly induced (30 seconds) by inflammatory mediators such as bradykinin (BK) or prokineticin 2 (PK2), followed by fixation with 4% paraformaldehyde and 4% sucrose in phosphate buffered saline (PBS, 50% dilution), staining for PKCε, and quantification of the number of neurons in which translocation is observed. CR4056 was pre-applied in the culture medium for 10 min or co-applied with the stimulus. After fixation, cells were permeabilized with 0.2% Triton X-100 (Sigma-Aldrich, Milan, Italy) and exposed overnight to a rabbit polyclonal antibody highly specific for PKCε. After extensive rinsing, PKCε was visualized with a secondary antibody (1:200 dilution Alexa Fluor 488 goat anti-rabbit, Thermo Fisher Scientific, Monza, Italy) applied for 2-4 hours at room temperature in the dark Cells showing PKCε translocation were detected with a confocal microscope (Leica SP2, Leica, Switzerland) by measuring fluorescence intensity along a line drawn through the cytoplasm and the membrane, thus avoiding the nucleus. Neurons in which fluorescence intensity at the plasma membrane throughout the cell was 1.5-fold or higher than the mean cytoplasmic intensity were considered positive.

### RESULTS

CR4056 dose-dependently inhibited PKCε translocation obtained with either BK or PK2 with IC₅₀ values respectively of 0.20 and 0.17 µM. When CR4056 was applied for 10 minutes, the dose-response curves approached saturation at about 10µM. This concentration was tested at different time intervals to investigate the kinetics of CR4056 effect. Prolonging (up to 24 hours) or reducing (10 seconds) the preincubation time did not change the extent of the effect. Then the time necessary to wash off the effect of this drug was analyzed. In Figure 1A and B, PKCε translocation is reported at different timepoints after CR4056 (10µM): first, just after the 10 minute application and then, after repeated, long lasting washes with large volumes of culture medium (DMEM + 10% FBS, 37°C) expected to remove any trace of CR4056 from the extracellular environment (washout). The effect of CR4056 remained unchanged for up to 1 hour after washout, then it slowly decreased and was completely reversed in 3-4 hours. The sensitivity of the PKCε translocation assay to idazoxan was then tested. Idazoxan was pre-applied for 10 minutes at high concentrations (10 and 100 µM), toward 1 µM CR4056, a concentration inducing submaximal block of PKCε translocation. Both concentrations of idazoxan were completely ineffective.

### CONCLUSIONS

The above reported results showed that CR4056 efficiently blocked PKCε translocation in neurons challenged with pro-inflammatory stimuli, that CR4056 effect had a fast onset, but it was very slowly removed from the cells, and that the pathway used by CR4056 to achieve this effect was idazoxan resistant demonstrating the involvement of non-classical I2 receptors. This peculiar mechanism of CR4056 anti-inflammatory activity in neurons, could contribute to the overall efficacy of this product in neurodegenerative processes and in cognition impairment, besides its established 12-driven effects.

### EXAMPLE 3: IN VIVO EFFECT OF 2-PHENYL-6-(1H-IMIDAZOL-1-YL) QUINAZOLINE (CR4056) ON MICROGLIA ACTIVATION IN CFA INFLAMMATORY MODEL

### METHODS

Microglial cells activation was evaluated by immunofluorescence staining, measuring the expression of ionized calcium-binding adapter molecule 1 (lba-1) in ipsilateral L5 spinal cord in complete Freund's adjuvant (CFA) model.

Monolateral inflammation was induced by injecting 100 µL CFA (1 mg/mL diluted 1:1 with saline) into the plantar surface of the right hind paw of rats.

CR4056 (6 mg/kg, os) was administered 72 hours post-CFA and after 90 minutes animals were deeply anesthetized with an overdose of urethane (1.5 g·kg-1, i.p.) and then transcardially perfused with 250 mL 0.9% saline containing 1% heparin (5000 UI·mL-1), followed by 500 mL 10% formalin (i.e. 4% paraformaldehyde, Bio-Optica Spa, Milan, Italy). The L5 segment of the spinal cord was harvested, post-fixed overnight at 4°C and embedded in paraffin blocks for sectioning. Transverse sections of the spinal cord were sliced with a fully automated rotary microtome at 5 µm thickness, mounted on poly-L-lysine coated slides and then processed for immunofluorescence. Antigen retrieval was performed using 10 mM citrate buffer (pH 6.0) for 20 minutes at 90°C. The sections were blocked with 10% normal horse serum in PBS containing 0.3% triton-X for 90 minutes at room temperature, and then incubated overnight at 4°C with rabbit anti-ionized calcium-binding adapter molecule 1 (lba1) primary polyclonal antibody (1:350; Wako Chemicals, Neuss, Germany #019-19741). For secondary detection, sections were incubated for 1 hours at room temperature with Alexa-Fluor 488 donkey anti-rabbit secondary antibody (1:400; Thermo Fisher Scientific, Waltham, MA, USA #A-21206). The slides were mounted with FluoroShield mounting medium with 4',6-diamidino-2-phenylindole or DAPI (Sigma-Aldrich, Milan, Italy #F6057) to counterstain the nuclei. Spinal cord sections were visualized with Invitrogen EVOS FL Auto Cell Imaging System (Thermo Fisher Scientific, Waltham, MA, USA). The contralateral side of each section was identified with a small cut in the ventral horn. Laminae I-III of the dorsal horns were identified taking as reference the rat brain atlas of Paxinos and Watson (Paxinos and Watson, 1982). For each section, a representative image of both the ipsilateral and the contralateral dorsal horn (laminae I-III) of the L5 spinal cord was captured at 20x magnification, using consistent exposure times across all sections, and then analysed. Iba1-positive microglial cells displaying an amoeboid/activated state (i.e. clearly swollen cell bodies with reduced processes) were manually counted. Results are expressed as the percentage of the ipsilateral/contralateral ratio of the number of Iba1-positive microglial cells displaying an activated state. For each animal (n=5 per group) 6 non-consecutive sections were analysed and results were averaged.

### RESULTS

The CFA-induced arthritic rat model is a paradigm of chronic inflammatory pain. Intraplantar injection of CFA resulted in increased sensitivity to noxious heat as well as heightened sensitivity to mechanical tactile stimulation in peripheral tissue injury, and induced a significant increase of the ratio of Iba1-positive, morphologically activated microglial cells, i.e. microglia activation, in laminae I-III of the L5 spinal cord ipsilateral vs. contralateral dorsal horns.

CR4056, after single administration (6 mg/kg, os), completely restored the basal conditions of microglia previously activated by CFA treatment (Figure 2).

### CONCLUSIONS

During the course of several diseases, microglia cells lose their homeostatic molecular signature and functions and become chronically inflamed and elicit detrimental effects. This is evident for neurodegenerative diseases, including Alzheimer's disease, amyotrophic lateral sclerosis, multiple sclerosis, Parkinson's disease, but also ageing and autism spectrum disorder (Butovsky O, Nature Rev Neuroscience 2018, Henstridge CM, Frontiers in Cellular Neuroscience, 2019; Wes PD, Glia 2016; Salter, MW, Nat. Med., 2017) and chronic pain (Malcangio M, Pain, 2016).

In this animal model CR4056 significantly reduced microglia activation, as highlighted by the reduction of Iba1-positive cells, thus contributing to the neuroprotection.

### EXAMPLE 4: IN VIVO EFFECT OF 2-PHENYL-6-(1H-IMIDAZOL-1-YL) QUINAZOLINE (CR4056) ON A MODEL OF SCOPOLAMINE-INDUCED MEMORY IMPAIRMENT: PASSIVE AVOIDANCE

### METHODS

Scopolamine, a nonselective muscarinic receptor antagonist, produces a blocking of the activity of the muscarinic acetylcholine receptor, and the concomitant appearance of transient cognitive amnesia and electrophysiological changes, which resemble those observed in Alzheimer's disease. Therefore, scopolamine administration may be considered as a psychopharmacological model of Alzheimer's disease (Lenz RA, Psychopharmacology (Berl), 2012).

Passive avoidance is a behavioural model of memory impairment.

The apparatus consisted of a two-compartment box: light and dark compartments separated by a door. The rat was placed in the center of the light compartment, with the door between two compartments closed. After 4 seconds, the door was opened and the latency time, i.e., the time taken by the rat to enter all four paws into the dark compartment was recorded.

Once the rat moved completely in the dark compartment, the door was closed and immediately a mild foot shock was delivered through the grid floor. Thus, during the initial phase the animal learned that the moving to the dark compartment had negative consequences.

Forty-height hours after training, the rat was placed in the light compartment and the same procedure of the training was followed except that no shock was applied to the grid floor. Memory performance was positively correlated with the latency to escape from the light compartment.

To induce memory impairment, scopolamine (1mg/kg sc) was administered 30 minutes before learning trial.

CR4056 (10 mg/kg bid) was administered orally immediately after learning trial, in order to avoid possible bias due to its analgesic effect.

### RESULTS

Figure 3 shows that, at the test day, sham animals (not treated with scopolamine) increased significantly the latency time to escape from light compartment, compared to training. In animals treated with scopolamine there was no increment of latency time.

CR4056 10 mg/kg bid counteracted the memory impairment (shown as increased escape latency time) induced by scopolamine.

The escape latency time in sham animals treated with CR4056 was not different from its vehicle (methylcellulose, MC).

### CONCLUSIONS

CR4056 was able to reverse the memory impairment in a pharmacological model of Alzheimer's disease and dementia.

### EXAMPLE 5: EFFECT OF 2-PHENYL-6-(1H-IMIDAZOL-1-YL)QUINAZOLINE (CR4056) ON SCOPOLAMINE-INDUCED MEMORY IMPAIRMENT BEHAVIOURAL MODEL IN THE RAT: MORRIS WATER MAZE

### METHODS

The Morris water maze test was used to evaluate cognitive function distinct in two phases: first, the acquisition and spatial localization of a hidden platform and, subsequently, the processing, consolidating, retaining and then retrieving the acquired information to successfully locate the platform to escape the water.

Place navigation required the rats to learn to swim from any starting position to the escape platform, thereby acquiring a long-term memory of the platform's spatial location. Animals were placed into various quadrants of the pool and the time elapsed and the distance traversed to reach the hidden platform was recorded. Various objects were placed in the testing room so that the animals used these visual cues as a means of navigating in the Maze. After repeated entry into the Maze, the animals became more and more efficient at locating the platform, thus escaping the water by learning the location of the platform relative to the distal visual cues.

To induce memory impairment, scopolamine was administered (1 mg/kg, sc) 30 minutes before the trial, whereas CR4056 was administered (20 mg/kg, os) 60 minutes before the trial. Four trials were conducted over four days and the final latency time reported was the mean over all days.

### RESULTS

CR4056 20 mg/kg reverted the impairment of memory induced by scopolamine. As reported in Figure 4, rats treated with scopolamine need more time to locate the hidden platform in comparison with control group. Coadministration of CR4056 with scopolamine significantly reduced the time taken to get to the platform respect to the animals treated with scopolamine alone.

### CONCLUSIONS

The treatment with CR4056 reverted the impairment of mnemonic ability that characterized the animal model of dementia obtained with scopolamine treatment.

### EXAMPLE 6: EFFECT OF 2-PHENYL-6-(1H-IMIDAZOL-1-YL)QUINAZOLINE (CR4056) IN A MOUSE MODEL OF SCOPOLAMINE-INDUCED MEMORY IMPAIRMENT: NOVEL OBJECT RECOGNITION

### METHODS

The novel object recognition test was used to evaluate cognitive function based on the ability of mice to recognize various object proposed in the arena test. During the training phase a pair of identical objects were placed in the cage test. 24 hours after the first phase, during the testing phase one of the two object was replaced by a new different one. Time of exploration of each object was recorded in training and testing phase. The typical approach of mice was a similar exploration of the objects in training phase and a preference for the new object during the testing phase. The administration of substances that can worsen cognitive performance (i.e. scopolamine) caused the deletion of reminder of the old object and an equal exploration of 2 different objects. Scopolamine 1 mg/kg was administered ip 20 minutes before the training, whereas CR4056 6 and 20 mg/kg was administered os 40 minutes before the training.

### RESULTS

CR4056 6 and 20 mg/kg increased the time of exploration of the new object (preference for) during the testing phase. Mice treated with scopolamine alone showed no preference for the new object (Figure 5).

### CONCLUSIONS

CR4056 dose dependently improved the mice mnemonic performance in this pharmacological model of dementia induced by scopolamine, confirming previously results obtained in rats.

### EXAMPLE 7: EFFECT OF 2-PHENYL-6-(1H-IMIDAZOL-1-YL)QUINAZOLINE (CR4056) IN TRANSGENIC 5XFAD MICE MODEL OF ALZHEIMER'S DISEASE: NOVEL OBJECT RECOGNITION

### METHODS

Transgenic 5XFAD mice, overexpressing the human amyloid precursor protein (APP695) with Swedish (K670N/M671L), Florida (1716V) and London (V717I) familial Alzheimer's disease (FAD) mutations, as well as human Presenilin1 (PS1) with M146L and L286V FAD mutations were used as Alzheimer's disease model. 6 months old female transgenic 5XFAD mice and wild-type controls (WT) were treated orally (gavage) with 30 mg/kg of CR4056 or vehicle once a day for a period of 10 days (n= 4 WT/Vehicle, 8 WT/CR4056, 2 5XFAD/Vehicle, and 3 5XFAD/CR4056). Following repeated treatments, hippocampal-dependent memory was tested using the novel object recognition task. The object recognition test measures working and spatial memory. In the training phase, animals were placed in the arena with objects constructed from large plastic bricks. In the testing phase, one object was replaced by a novel object. Animals were returned in the arena and allowed to explore. Exploration of objects was observed and recorded. Mice should recognize that a new object has been placed and therefore explore this object for longer time.

### RESULTS

On the test day, the 5XFAD vehicle group showed cognitive deficits, whereas 5XFAD animals treated with CR4056 showed a significant improvement in the working and spatial memory (Figure 6).

### CONCLUSIONS

5XFAD mouse is a model of Alzheimer's disease characterized by intraneuronal Aβ aggregates, neurodegeneration, neuron loss, impaired memory accompanied with glial activation (Oakley H, The journal of Neuroscience, 2006; Mirzaei N, Glia 2006).

In this transgenic model, CR4056 significantly improved the mnemonic performance, in agreement with results obtained in the pharmacological model of dementia induced by scopolamine.

### EXAMPLE 8: BRAIN PENETRATION OF 2-PHENYL-6-(1H-IMIDAZOL-1-YL)QUINAZOLINE (CR4056)

### METHODS

CR 4056 was determined in rat plasma and in rat brain and plasma using a LC/MS/MS method. Male Sprague Dawley rats, Harlan, were treated orally with a CR 4056 suspension (Methocel 0.5%) at the dose of 30 mg/kg. Oral administration was performed by gastric gavage to 3 animals per group (5 ml/kg). The compound was detected in plasma and in brain 1 hour after the treatment.

Each rat brain was weighted and cut in half. Each cerebral hemisphere was weighted and homogenized with two different methodologies. One was liquidization by Ultra turrax tube drive (IKA), adding an aliquot of buffer solution (10mM ammonium formate pH 3.5) three times the weight of the hemisphere. Then 30 µl of the extract solution were added to 0.25 ml of methanol in a 1.5 ml eppendorf tube, vortex-mixed and centrifugated for 5 min at 13500 rpm at 4°C. the supernatant was transferred to a 96-deep well plates 2mL round (Axygen) and injected into the LC/MS/MS system. The second one was cryogenic grinding with Mikro Dismembrator (Sartorius). Each cerebral hemisphere was weighted, put in a 20 mL teflon vessel with a steel sphere (7,85 g/ml dia 10 mm) and frozen by immersion in liquid nitrogen for 5 minutes. The deep frozen brain was ground with Mikro Dismembrator at 2500 rpm for 30 sec, the freezing/grinding cycle was repeated twice. A portion of powder was accurately weighted (ca. 10 mg) in a 1.5 ml eppendorf tube, 0.25 ml of methanol were then added. After vortex-mixing and centrifugation for 5 min at 13500 rpm at 4°C, the supernatant was placed in a 96-deep well plates 2mL round (Axygen) and injected into the LC/MS/MS system. Both methods used for homogenization gave similar results showing that the extraction efficiency was similar for both methods.

### RESULTS

Individual plasma and brain levels and brain plasma ratio after oral administration (30 mg/kg) of CR 4056 suspension to male Sprague Dawley rats in fasted conditions (rats were scarified 60 minutes after the PO administration) are shown in the following table.

| Rat | Actual | Route | Rat | Actual | Plasmatic | Mean Brain | Brain |
|---|---|---|---|---|---|---|---|
| ID | dose | | weight | time | Concentration | Concentration | /plasma |
| | (mg) | | (kg) | (h) | (ng/mL) | (ng/g) | ratio |
| 1 | 6.6 | PO | 0.216 | 1 | 2860 | 33580 | 11.7 |
| 2 | 6.6 | PO | 0.212 | 1 | 2860 | 35492 | 12.4 |
| 3 | 6.6 | PO | 0.212 | 1 | 2430 | 27745 | 11.4 |
| **Mean** | **6.6** | **PO** | **0.213** | **1** | **2717** | **32272** | **11.8** |

This experiment hence demonstrated that CR4056 was capable to effectively enter into the brain, thus having an effect directly in the central nervous system (CNS).

## Claims

1. A compound of formula 2-phenyl-6-(1H-imidazol-1-yl)quinazoline or a pharmaceutically acceptable salt thereof for use in the treatment of a neurodegenerative disease, wherein the neurodegenerative disease is a disease selected from the group consisting of Alzheimer's disease, Lewy body dementia, frontotemporal dementia, amyotrophic lateral sclerosis, Huntington disease, prion diseases and HIV-associated dementia.

2. The compound for use according to claim 1, wherein the neurodegenerative disease is Alzheimer's disease.

3. The compound for use according to claim 1 or claim 2, wherein the pharmaceutically acceptable salt of 2-phenyl-6-(1H-imidazol-1-yl)quinazoline is a salt chosen from hydrochloride, hydrobromide, hydrogensulphate and sulphate, maleate, fumarate, oxalate, methanesulfonate, succinate, ascorbate, tartrate, acetate, salicylate, citrate, aspartate, ethylenediaminotetraacetate, benzoate and glutamate.

4. A pharmacological composition comprising the compound of formula 2-phenyl-6-(1H-imidazol-1-yl)quinazoline or a pharmaceutically acceptable salt thereof and a carrier for use in the treatment of a neurodegenerative disease, wherein the neurodegenerative disease is a disease selected from the group consisting of Alzheimer's disease, Lewy body dementia, frontotemporal dementia, amyotrophic lateral sclerosis, Huntington disease, prion diseases and HIV-associated dementia.

5. The composition for use according to claim 4, wherein the neurodegenerative disease is Alzheimer's disease.

6. The composition for use according to claim 4 or 5, wherein 2-phenyl-6-(1 H-imidazol-1-yl)quinazoline (CR4056) or a pharmaceutically acceptable salt is in an amount from 15 to 250 mg with respect to the unitary dosage form, resulting in a daily intake from 15 to 500 mg.

7. The composition for use according to anyone of claims 4-6 wherein the composition comprises at least one further drug.

8. A combined pharmaceutical preparation comprising 2-phenyl-6-(1H-imidazol-1-yl)quinazoline (CR4056) or a pharmaceutically acceptable salt thereof and at least one of NMDA (N-methyl-D-aspartic acid) receptor antagonists and/or acetylcholinesterase inhibitors for simultaneous, sequential or separated use in the treatment of a neurodegenerative disease, wherein the neurodegenerative disease is a disease selected from the group consisting of Alzheimer's disease, Lewy body dementia, frontotemporal dementia, amyotrophic lateral sclerosis, Huntington disease, prion diseases and HIV-associated dementia.

9. The combined pharmaceutical preparation for use according to claim 8, wherein the neurodegenerative disease is Alzheimer's disease.

## Patentansprüche

1. Verbindung der Formel 2-Phenyl-6-(1H-imidazol-1-yl)chinazolin oder ein pharmazeutisch verträgliches Salz davon zur Verwendung bei der Behandlung einer neurodegenerativen Erkrankung, wobei die neurodegenerative Erkrankung eine Erkrankung ist, die ausgewählt ist aus der Gruppe bestehend aus Alzheimer-Krankheit, Lewy-Körper-Demenz, frontotemporaler Demenz, amyotropher Lateralsklerose, Huntington-Krankheit, Prionenerkrankungen und HIV-assoziierter Demenz.

2. Verbindung zur Verwendung nach Anspruch 1, wobei die neurodegenerative Erkrankung die Alzheimer-Krankheit ist.

3. Verbindung zur Verwendung nach Anspruch 1 oder Anspruch 2, wobei das pharmazeutisch annehmbare Salz von 2-Phenyl-6-(1H-imidazol-1-yl)chinazolin ein Salz ist, das ausgewählt ist aus Hydrochlorid, Hydrogenbromid, Hydrogensulfat und Sulfat, Maleat, Fumarat, Oxalat, Methansulfonat, Succinat, Ascorbat, Tartrat, Acetat, Salicylat, Citrat, Aspartat, Ethylendiamintetraacetat, Benzoat und Glutamat.

4. Pharmakologische Zusammensetzung, umfassend die Verbindung der Formel 2-Phenyl-6-(1H-imidazol-1-yl)chinazolin oder ein pharmazeutisch annehmbares Salz davon und einen Träger zur Verwendung bei der Behandlung einer neurodegenerativen Erkrankung, wobei die neurodegenerative Erkrankung eine Erkrankung ist, die ausgewählt ist aus der Gruppe aus Alzheimer-Krankheit, Lewy-Körper-Demenz, frontotemporaler Demenz, amyotropher Lateralsklerose, Huntington-Krankheit, Prionenerkrankungen und HIV-assoziierter Demenz.

5. Zusammensetzung zur Verwendung nach Anspruch 4, wobei die neurodegenerative Erkrankung die Alzheimer-Krankheit ist.

6. Zusammensetzung zur Verwendung nach Anspruch 4 oder 5, wobei 2-Phenyl-6-(1H-imidazol-1-yl)chinazolin (CR4056) oder ein pharmazeutisch annehmbares Salz in einer Menge von 15 bis 250 mg, bezogen auf die Einheitsdosierungsform, vorliegt, was zu einer täglichen Aufnahme von 15 bis 500 mg führt.

7. Zusammensetzung zur Verwendung nach einem der Ansprüche 4 bis 6, wobei die Zusammensetzung mindestens ein weiteres Arzneimittel enthält.

8. Kombiniertes pharmazeutisches Präparat, umfassend 2-Phenyl-6-(1H-imidazol-1-yl) Chinazolin (CR4056) oder ein pharmazeutisch annehmbares Salz davon und mindestens einen von NMDA (N-Methyl-D-Asparaginsäure)-Rezeptorantagonisten und/oder Acetylcholinesterase-Inhibitoren zur gleichzeitigen, aufeinanderfolgenden oder getrennten Verwendung bei der Behandlung einer neurodegenerativen Erkrankung, wobei die neurodegenerative Erkrankung eine Erkrankung ist, die ausgewählt ist aus der Gruppe bestehend aus Alzheimer-Krankheit, Lewy-Körper-Demenz, frontotemporaler Demenz, amyotropher Lateralsklerose, Huntington-Krankheit, Prionenerkrankungen und HIV-assoziierter Demenz.

9. Kombiniertes pharmazeutisches Präparat zur Verwendung nach Anspruch 8, wobei die neurodegenerative Erkrankung die Alzheimer-Krankheit ist.

## Revendications

1. Composé de formule de quinazoline 2-phényl-6-(1H-imidazol-1-yl) ou un sel pharmaceutiquement acceptable de celui-ci pour une utilisation dans le traitement d'une maladie neurodégénérative, dans lequel la maladie neurodégénérative est une maladie choisie dans le groupe constitué par la maladie d'Alzheimer, la démence à corps de Lewy, la démence frontotemporale, la sclérose latérale amyotrophique, la maladie de Huntington, les maladies à prions et la démence associée au VIH.

2. Composé pour une utilisation selon la revendication 1, dans lequel la maladie neurodégénérative est la maladie d'Alzheimer.

3. Composé pour une utilisation selon la revendication 1 ou la revendication 2, dans lequel le sel pharmaceutiquement acceptable de quinazoline 2-phényl-6-(1H-imidazol-1-yl) est un sel choisi parmi le chlorhydrate, le bromhydrate, l'hydrogénosulfate et le sulfate, le maléate, le fumarate, l'oxalate, le méthanesulfonate, la succinate, l'ascorbate, le tartrate, l'acétate, le salicylate, le citrate, l'aspartate, l'éthylènediaminotétraacétate, le benzoate et le glutamate.

4. Composition pharmacologique comprenant le composé de formule quinazoline 2-phényl-6-(1H-imidazol-1-yl) ou un sel pharmaceutiquement acceptable de celui-ci et un support pour une utilisation dans le traitement d'une maladie neurodégénérative, dans laquelle la maladie neurodégénérative est une maladie choisie dans le groupe constitué par la maladie d'Alzheimer, la démence à corps de Lewy, la démence frontotemporale, la sclérose latérale amyotrophique, la maladie de Huntington, les maladies à prions et la démence associée au VIH.

5. Composition pour une utilisation selon la revendication 4, dans laquelle la maladie neurodégénérative est la maladie d'Alzheimer.

6. Composition pour une utilisation selon la revendication 4 ou 5, dans laquelle la quinazoline 2-phényl-6-(1H-imidazol-1-yl) (CR4056) ou un sel pharmaceutiquement acceptable est en une quantité de 15 à 250 mg par rapport à la forme posologique unitaire, ce qui donne une prise quotidienne de 15 à 500 mg.

7. Composition pour une utilisation selon l'une quelconque des revendications 4 à 6, dans laquelle la composition comprend au moins un autre médicament.

8. Préparation pharmaceutique combinée comprenant de la quinazoline 2-phényl-6-(1 H-imidazol-1-yl) (CR4056) ou un sel pharmaceutiquement acceptable de celui-ci et au moins un des antagonistes de récepteur et/ou des inhibiteurs d'acétylcholinestérase NMDA (acide N-méthyl-D-aspartique) pour une utilisation simultanée, séquentielle ou séparée dans le traitement d'une maladie neurodégénérative, dans laquelle la maladie neurodégénérative est une maladie choisie dans le groupe constitué par la maladie d'Alzheimer, la démence à corps de Lewy, la démence frontotemporale, la sclérose latérale amyotrophique, la maladie de Huntington, les maladies à prions et la démence associée au VIH.

9. Préparation pharmaceutique combinée pour une utilisation selon la revendication 8, dans laquelle la maladie neurodégénérative est la maladie d'Alzheimer.
